# EUROPEAN PATENT APPLICATION

(11) **EP 4 212 610 A1**
(43) Date of publication of application: **19.07.2023**
(21) Application number: 21866674.1
(22) Date of filing: 03.09.2021
(51) Int. Cl.: C12M 1/00, C12N 15/11, C12Q 1/6844, C12Q 1/6876

(54) **DESIGN METHOD FOR DIVIDING PRIMER PAIRS INTO REACTION CONTAINERS, METHOD OF AMPLIFYING TARGET NUCLEIC ACID, TUBE SET, LIST OF PRIMER PAIRS, AND PROGRAM FOR DIVIDING PRIMER PAIRS INTO REACTION CONTAINERS**

(30) Priority: 11.09.2020 JP 2020152969
(71) Applicant: FUJIFILM Corporation, Tokyo 106-8620 (JP)
(72) Inventor: NAGASE, Masaya, Ashigarakami-gun, Kanagawa 258-8577 (JP); ASANUMA, Masayo, Ashigarakami-gun, Kanagawa 258-8577 (JP); TSUJIMOTO, Takayuki, Ashigarakami-gun, Kanagawa 258-8577 (JP); GOTO, Kengo, Ashigarakami-gun, Kanagawa 258-8577 (JP)
(74) Representative: Klunker IP Patentanwälte PartG mbB
(86) International application number: PCT/JP2021/032469
(87) International publication number: WO 2022/054715

(57) **Abstract**

Provided is a design method for dividing primer pairs into reaction containers, the design method showing an optimum division example. The design method for dividing primer pairs into reaction containers has a design step of, for a plurality of target nucleic acids, designing a plurality of primer pairs each composed of two types of primers, an evaluation step of evaluating non-specific amplification inducibility between the primer pairs, and an assignment step of performing an assignment to the reaction containers, based on the non-specific amplification inducibility, such that primer pairs having the non-specific amplification inducibility are not present in the same reaction container. The assignment step has a graph generation step of generating a graph having the primer pairs as vertices and non-specific amplification inducibility as an edge or a data structure equivalent to the graph, a coloring step of applying a solution to a graph coloring problem or the like to the graph to perform coloring such that the vertices adjacent to each other have different colors, and an association step of associating the plurality of colors with the reaction containers to associate the primer pair with the reaction containers of the corresponding colors.

## Description

### BACKGROUND OF THE INVENTION

### 1. Field of the Invention

The present invention relates to a design method for dividing primer pairs into reaction containers, a method for amplifying target nucleic acids, a tube set, a list of primer pairs, and a program for dividing primer pairs into reaction containers. In particular, the present invention relates to a design method for dividing primer pairs into reaction containers in order to efficiently amplify a large number of genes, a method for amplifying target nucleic acids, a tube set, a list of primer pairs, and a program for dividing primer pairs into reaction containers.

### 2. Description of the Related Art

In recent years, the importance of gene analysis has been increasing in studies in the field of biotechnology. Genes are generally nucleic-acid base sequences, and analysis of genes is conducted by a method for reading a base sequence as it is or a method for reading a base sequence after amplification with the aim of conducting a measurement with a very small amount of sample or reducing the cost due to screening. Polymerase chain reaction (PCR) is commonly performed as a method for amplifying a gene (base sequence). In PCR, primers which are base sequences complementary to a target nucleic acid are introduced to amplify the target nucleic acid in a chain reaction. Furthermore, multiplex PCR (MPCR) is performed as a method for simultaneously amplifying a plurality of genes.

However, the MPCR method has a problem in that target nucleic acids cannot be amplified by a plurality of inhibition factors, for example, in the case where side reaction occurs between primers to generate a noise component called a primer dimer, which is not included in a target sample, or in the case where a primer is bound to a nucleic acid derived from a gene other than a nucleic acid derived from a target gene.

To solve this problem, the design of primers has been devised. However, for example, when a gene having only a very short base sequence, which is called a micro-RNA (miRNA), is to be a target, there is a limitation in the adjustment with the primer design because of low design flexibility of primers. In such a case, it is also conceivable that primers are separately assigned to reaction containers (tubes) to thereby prevent the primers from binding to a nucleic acid other than a nucleic acid derived from a target gene. This is a kind of combinational optimization problem that how a gene and primers are combined; however, sufficient consideration has not been given as to how the optimization should be performed.

For example, JP4436039B discloses a method for simultaneously amplifying target nucleic acids, in which a concentration of a primer is adjusted before reaching the reaction plateau (the saturation of amplification). JP5709897B discloses that while nested PCR is performed, an adjustment is performed at a number of cycles at which a false positive signal is not generated. JP4879975B discloses a method having a step of separating primers into different reaction containers in order to amplify a short nucleic acid.

Meanwhile, in the mathematical studies, there is a field called a graph, in particular, a field called a graph coloring problem. This is a combinational optimization problem in which an undirected graph G is colored such that adjacent vertices have colors different from each other with just K types of colors. Also in the field of gene measurement, US6074831A discloses that a graph coloring problem is used for a nucleic acid amplification technology and discloses, as one example thereof, an example in which tubes are partitioned with a size of an amplicon. It is disclosed that when the amplification of a gene is checked by electrophoresis, the positions of bands vary depending on the size of a gene; therefore, an amplified gene can be identified by preventing the same size from being assigned to the identical tube.

### SUMMARY OF THE INVENTION

The methods described in JP4436039B and JP5709897B each propose optimization under a wet condition, and no dry condition was investigated. JP4879975B does not consider how respective primers are assigned to the reaction containers. The method described in US6074831A describes no embodiment considering non-specificity between primers, that is, the phenomenon that non-specific amplification is induced when a different type of introduced primer pair is bridged.

Thus, none of the methods described in JP4436039B, JP5709897B, JP4879975B, and US6074831A examine that when a plurality of target nucleic acids are simultaneously amplified, primers are divided into reaction containers considering that side reaction occurs between primers to generate a noise component that is not included in a target sample or in consideration of non-specificity that a primer is bound to a nucleic acid derived from a gene other than a nucleic acid derived from a target gene.

The present invention has been made in view of the circumstances described above. An object of the present invention is to provide a design method for dividing primer pairs into reaction containers, the design method showing a division example that is optimum for dividing primer pairs into reaction containers when a plurality of target nucleic acids are simultaneously amplified using a plurality of reaction containers, a method for amplifying target nucleic acids, a tube set, a list of primer pairs, and a program for dividing primer pairs into reaction containers.

To achieve the object of the present invention, a design method for dividing primer pairs into reaction containers according to the present invention is a design method for dividing primer pairs into a plurality of reaction containers to simultaneously amplify a plurality of target nucleic acids, the design method having a design step of, for each of the plurality of target nucleic acids, designing a primer pair composed of two types of primers that complementarily form a pair to design a plurality of primer pairs; an evaluation step of evaluating non-specific amplification inducibility between a primer constituting a primer pair that forms a pair with one target nucleic acid and a primer constituting a primer pair that forms a pair with another target nucleic acid; and an assignment step of performing an assignment to the plurality of reaction containers, based on the non-specific amplification inducibility evaluated in the evaluation step, such that primer pairs including primers having the non-specific amplification inducibility are not present in the same reaction container, wherein the assignment step has a graph generation step of generating a graph having the primer pairs as vertices and non-specific amplification inducibility between primers constituting the primer pairs as an edge or a data structure equivalent to the graph, a coloring step of applying a solution to a graph coloring problem to the graph or applying a problem equivalent to a graph coloring problem and a solution thereto to the data structure equivalent to the graph to color the vertices in a plurality of conceptual colors such that the vertices adjacent to each other with the edge therebetween have different colors, and an association step of associating the plurality of conceptual colors colored in the coloring step with the reaction containers to associate the primer pairs corresponding to the vertices with the reaction containers of the corresponding colors.

According to another aspect of the present invention, the graph generation step preferably has an extraction step of specifying and extracting, from the primer pairs, a primer set consisting of primer pairs having high similarity, a selection step of selecting one or more representative primer pairs from the primer set, and a deletion step of excluding the target nucleic acids that form pairs with primer pairs that have not been selected as the representative primer pairs in the selection step among the primer pairs included in the primer set and deleting, in the graph, vertices of the primer pairs corresponding to the excluded target nucleic acids and an edge in contact with the vertices.

According to another aspect of the present invention, in the coloring step, the number of vertices colored in each color is preferably equalized.

According to another aspect of the present invention, preferably, the assignment step has, after the graph generation step, an input step of inputting the number k of the plurality of reaction containers, and a saving step of saving the vertices with a number of the edges of (k - 1) or less from the graph, and the assignment step has, after the saving step followed by the coloring step, a return step of returning the saved vertices with a number of the edges of (k - 1) or less.

According to another aspect of the present invention, in the return step, return is preferably performed such that the number of the primer pairs divided into each of the reaction containers is equalized.

According to another aspect of the present invention, in the return step, return is preferably performed such that the number of colors of each of the plurality of conceptual colors colored in the coloring step is equalized.

According to another aspect of the present invention, the assignment step preferably has, after the graph generation step, a division step of dividing the graph into connected graphs that are independent from each other, and an integration step of, after the coloring step being performed for the connected graphs, integrating the connected graphs to generate the graph.

According to another aspect of the present invention, preferably, the target nucleic acids are each a small RNA having a number of bases of 200 or less, one side of each of the primer pairs is a stem-loop primer, and in the evaluation step, when a complementarity score S is represented by S = m - u - 3d, where m represents the number of matches, u represents the number of mismatches, and d represents the number of insertions/deletions, inducibility of non-specific reaction between the primers is determined from: (A) for the stem-loop primer, the number of consecutive matches of bases on a 3'-end-side is 4 or more or the complementarity score S satisfies S > 5 and (B) for an ordinary primer, the complementarity score S satisfies S > 9, and when one of (A) and (B) is satisfied, the primers are determined to have non-specific amplification inducibility.

According to another aspect of the present invention, the target nucleic acids each preferably have a number of bases of 32 or less.

According to another aspect of the present invention, preferably, the coloring step employs a method based on a graph coloring problem, and coloring results are searched using ZDD.

According to another aspect of the present invention, preferably, the coloring step employs a method based on a graph coloring problem, and the coloring results are searched by, using ZDD, enumerating maximal independent sets on the graph and determining a combination that covers vertices of the graph with some or all of the enumerated maximal independent sets.

To achieve the object of the present invention, a method for amplifying target nucleic acids according to the present invention is a method for amplifying target nucleic acids, the method having a step of adding a sample including a plurality of target nucleic acids to a plurality of reaction containers; a step of adding, based on the above design method for dividing primer pairs into reaction containers, the primer pairs to the corresponding reaction containers; and a step of amplifying the target nucleic acids in the reaction containers.

To achieve the object of the present invention, a tube set according to the present invention is a tube set including a plurality of tubes for simultaneously amplifying a plurality of target nucleic acids having a number of bases of 32 or less, wherein each tube of the plurality of tubes includes, for each of the plurality of target nucleic acids, at least one primer pair composed of two types of primers that complementarily form a pair, one side of the primer pair is a stem-loop primer, and when two or more of the primer pairs are included in one of the tubes and a complementarity score S is represented by S = m - u - 3d, where m represents the number of matches, u represents the number of mismatches, and d represents the number of insertions/deletions, inducibility of non-specific reaction between the primers in the tube is determined from: (A) for the stem-loop primer, the number of consecutive matches of bases on a 3'-end-side is 4 or more or the complementarity score S satisfies S > 5 and (B) for an ordinary primer, the complementarity score S satisfies S > 9, and the tube set includes no primer pair that satisfies one of (A) and (B).

According to another aspect of the present invention, a total number of the plurality of target nucleic acids is preferably 50 or more.

According to another aspect of the present invention, the total number of the plurality of target nucleic acids is preferably 100 or more.

To achieve the object of the present invention, a list of primer pairs according to the present invention is a list of primer pairs divided into a plurality of groups for simultaneously amplifying a plurality of target nucleic acids having a number of bases of 32 or less, wherein each group of the plurality of groups includes, for each of the plurality of target nucleic acids, at least one primer pair composed of two types of primers that complementarily form a pair, one side of the primer pair is a stem-loop primer, and when two or more of the primer pairs are included in one of the groups and a complementarity score S is represented by S = m - u - 3d, where m represents the number of matches, u represents the number of mismatches, and d represents the number of insertions/deletions, inducibility of non-specific reaction between the primers in the group is determined from: (A) for the stem-loop primer, the number of consecutive matches of bases on a 3'-end-side is 4 or more or the complementarity score S satisfies S > 5 and (B) for an ordinary primer, the complementarity score S satisfies S > 9, and the list of primer pairs includes no primer pair that satisfies one of (A) and (B).

According to another aspect of the present invention, a total number of the plurality of target nucleic acids is preferably 50 or more.

According to another aspect of the present invention, the total number of the plurality of target nucleic acids is preferably 100 or more.

To achieve the object of the present invention, a program for dividing primer pairs into reaction containers according to the present invention is a program for dividing primer pairs into a plurality of reaction containers to simultaneously amplify a plurality of target nucleic acids that are each a small RNA having a number of bases of 200 or less, the program having a step of, for each of the plurality of target nucleic acids, designing a primer pair composed of two types of primers which complementarily form a pair and one of which is a stem-loop primer to design a plurality of primer pairs; a step of evaluating non-specific amplification inducibility between a primer constituting a primer pair that forms a pair with one target nucleic acid and a primer constituting a primer pair that forms a pair with another target nucleic acid; and a step of performing an assignment to the plurality of reaction containers, based on the non-specific amplification inducibility evaluated in the step of evaluating the non-specific amplification inducibility, such that primer pairs including primers having the non-specific amplification inducibility are not present in the same reaction container, wherein the step of performing an assignment has a step of generating a graph having the primer pairs as vertices and non-specific amplification inducibility between primers constituting the primer pairs as an edge or a data structure equivalent to the graph, a coloring step of applying a solution to a graph coloring problem to the graph or applying a problem equivalent to a graph coloring problem and a solution thereto to the data structure equivalent to the graph to color the vertices in a plurality of conceptual colors such that the vertices adjacent to each other with the edge therebetween have different colors, and a step of associating the plurality of conceptual colors colored in the coloring step with the reaction containers to associate the primer pairs corresponding to the vertices with the reaction containers of the corresponding colors, and in the step of evaluation, when a complementarity score S is represented by S = m - u - 3d, where m represents the number of matches, u represents the number of mismatches, and d represents the number of insertions/deletions, inducibility of non-specific reaction between the primers is determined from: (A) for the stem-loop primer, the number of consecutive matches of bases on a 3'-end-side is 4 or more or the complementarity score S satisfies S > 5 and (B) for an ordinary primer, the complementarity score S satisfies S > 9, and when one of (A) and (B) is satisfied, the primers are determined to have non-specific amplification inducibility.

According to the present invention, when a plurality of target nucleic acids are simultaneously amplified using a plurality of reaction containers, primer pairs can be separately added to the reaction containers by a division method optimum for inhibiting non-specific amplification between primers constituting the primer pairs added to the reaction containers. Accordingly, the amplification of the target nucleic acids and prevention of the amplification of nucleic acids other than the target nucleic acids can be achieved.

### BRIEF DESCRIPTION OF THE DRAWINGS

Fig. 1 is a block diagram illustrating the configuration of a primer division-designing apparatus;
Fig. 2 is a block diagram illustrating the configuration of a processing unit;
Fig. 3 is a flowchart illustrating a design method for dividing primers into reaction containers;
Fig. 4 is a flowchart illustrating steps of an assignment step;
Fig. 5 is a diagram for describing a state where a primer pair is bridged and amplified;
Fig. 6 is a diagram for describing an assignment step;
Fig. 7 is a diagram for describing a procedure for enumerating maximal independent sets of a graph example using ZDD;
Fig. 8 is a diagram for describing a procedure for enumerating maximal independent sets of a graph example using ZDD;
Fig. 9 is a diagram for describing a procedure for enumerating graph entire coverage by MIS using ZDD;
Fig. 10 is a flowchart illustrating a method for amplifying target nucleic acids;
Fig. 11 is a flowchart illustrating steps of an assignment step of a first modification;
Fig. 12 is an image diagram of a saving step of the first modification;
Fig. 13 is an image diagram of a division step of the first modification;
Fig. 14 is an image diagram of an integration step and a return step of the first modification;
Fig. 15 is a flowchart illustrating steps of a graph generation step of a second modification;
Fig. 16 is a flowchart for describing a design method for dividing primer pairs into reaction containers in Example 1; and
Fig. 17 is a graph illustrating an example of a subgraph coloring of a third graph in Example 1.

### DESCRIPTION OF THE PREFERRED EMBODIMENTS

Hereinafter, a design method for dividing primer pairs into reaction containers, a method for amplifying target nucleic acids, a tube set, a list of primer pairs, and a program for dividing primer pairs into reaction containers according to the present invention will be described with reference to the attached drawings. First, a description will be made of a primer division-designing apparatus for performing a design method for dividing primers into reaction containers according to this embodiment.

### Primer Division-Designing Apparatus

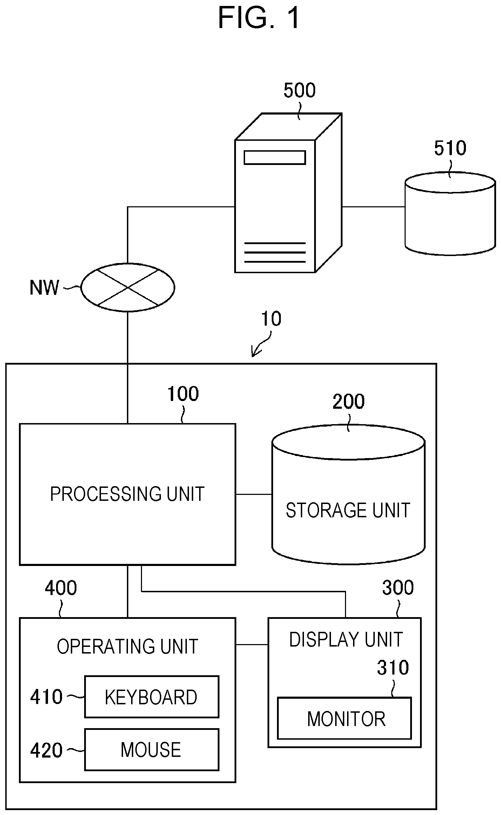

Fig. 1 is a block diagram illustrating the configuration of a primer division-designing apparatus (hereinafter, also simply referred to as a "designing apparatus") 10. The designing apparatus 10 is an apparatus for designing a division of primer pairs into a plurality of reaction containers in order to simultaneously amplifying a plurality of target nucleic acids and can be realized by using a computer. As illustrated in Fig. 1, the designing apparatus 10 includes a processing unit 100, a storage unit 200, a display unit 300, and an operating unit 400, and these units are connected to one another to transmit and receive necessary information. For these constituent elements, various installation forms may be employed, and the constituent elements may be installed at one position (e.g., in one housing or in one room) or may be connected to one another via a network installed at a separate position. The designing apparatus 10 is connected to an external server 500 and an external database 510 via a network NW, such as the internet, and can acquire information on, for example, an algorithm used for the evaluation of non-specific amplification inducibility in an evaluation step, as necessary. In addition, the designing apparatus 10 can acquire, in an assignment step, information on a data structure equivalent to a graph used when vertices are colored in a plurality of colors and a problem equivalent to a graph coloring problem and a solution thereto, and a program used for, for example, a search using ZDD (zero-suppressed BDD (binary decision diagram)). Configuration of Processing Unit

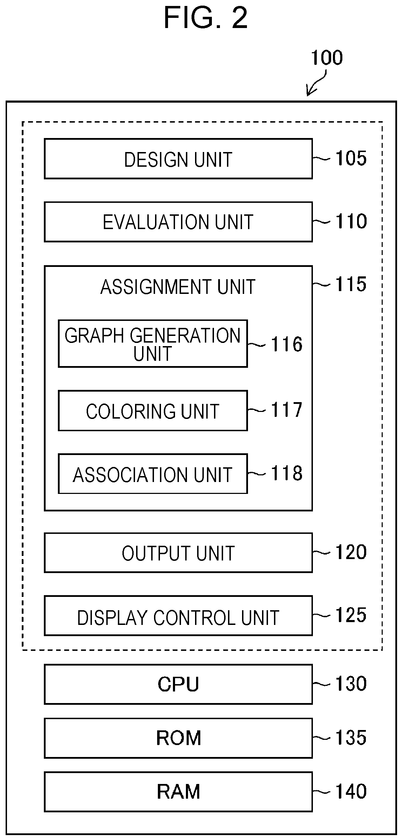

Fig. 2 is a block diagram illustrating the configuration of the processing unit 100. The processing unit 100 includes a design unit 105, an evaluation unit 110, an assignment unit 115, an output unit 120, a display control unit 125, a central processing unit (CPU) 130, a read only memory (ROM) 135, and a random access memory (RAM) 140.

For each target nucleic acid to be amplified, the design unit 105 designs a primer pair composed of two types of primers that complementarily form a pair. In this embodiment, a case where a plurality of target nucleic acids are simultaneously amplified is assumed, and a primer pair is designed for each of the plurality of target nucleic acids; therefore, a plurality of primer pairs are designed. The evaluation unit 110 evaluates non-specific amplification inducibility between primers constituting a primer pair designed in the design unit 105. A plurality of primer pairs are designed in the design unit 105, and non-specific amplification inducibility between primers constituting each of the primer pairs is evaluated. On the basis of the non-specific amplification inducibility evaluated in the evaluation unit 110, the assignment unit 115 assigns primer pairs to a plurality of reaction containers such that primer pairs that include primers having non-specific amplification inducibility are not present in the same reaction container.

The assignment unit 115 includes a graph generation unit 116, a coloring unit 117, and an association unit 118. The graph generation unit 116 generates a graph having the primer pairs designed in the design unit 105 as vertices, and a line connecting primer pairs determined, in the evaluation unit 110, to have non-specific amplification inducibility between primers as an edge. Alternatively, the graph generation unit 116 generates a data structure equivalent to the graph. The coloring unit 117 colors the vertices with regard to the graph or the data structure equivalent to the graph generated in the graph generation unit 116. In the coloring of the vertices, coloring is performed using a plurality of conceptual colors by applying a solution to a graph coloring problem or by applying a problem equivalent to a graph coloring problem and a solution thereto such that vertices adjacent to each other with an edge therebetween have different colors. Specifically, coloring is performed such that vertices corresponding to primer pairs having non-specific amplification inducibility have different colors. The association unit 118 associates the plurality of conceptual colors colored in the coloring unit 117 with the reaction containers to associate the primer pairs corresponding to the vertices with the reaction containers of the corresponding colors.

The output unit 120 outputs the plurality of primer pairs designed in the design unit 105. The output unit 120 further outputs the graph generated in the graph generation unit 116 and the graph colored in the coloring unit 117 of the assignment unit 115. The display control unit 125 controls the display of acquired information and processing results on a monitor 310. The processing of the design method for dividing primer pairs into reaction containers using these functions of the processing unit 100 will be described in detail below. The processing using these functions is performed under control by the CPU 130.

The above-described functions of the units of the processing unit 100 can be implemented by using various types of processors. The various types of processors include, for example, a CPU which is a general-purpose processor that executes software (program) to implement various functions. The above-described various types of processors further include a programmable logic device (PLD) which is a processor whose circuit configuration can be changed after manufacturing, such as a field programmable gate array (FPGA). Furthermore, the above-described various types of processors include, for example, a dedicated electric circuit which is a processor having a circuit configuration designed exclusively for executing specific processing, such as an application specific integrated circuit (ASIC).

The function of each unit may be implemented by one processor or may be implemented by a combination a plurality of processors. A plurality of functions may be implemented by one processor. A first example of implementing a plurality of functions by one processor is a pattern in which a combination of one or more CPUs and software constitutes one processor and this processor implements the plurality of functions, as represented by a computer such as a client or a server. A second example thereof is a pattern in which a processor that implements the functions of an entire system by one integrated circuit (IC) chip is used, as represented by a system on chip (SoC). In this manner, various functions are configured as a hardware structure by using one or more of the above various types of processors. Furthermore, the hardware structure of the various types of processors is, more specifically, electric circuitry formed by combining circuit elements such as semiconductor elements.

When the above-described processor or electric circuitry executes the software (program), a processor (computer)-readable code of the software to be executed is stored in a non-transitory recording medium, such as the ROM 135 (refer to Fig. 2), and the processor refers to the software. The software stored in the non-transitory recording medium includes a program for dividing primer pairs into reaction containers according to the present invention. The code may be recorded on a non-transitory recording medium, such as a magneto-optical recording apparatus or a semiconductor memory, instead of the ROM 135. In the processing using the software, the RAM 140 may be used as a temporary storage area, for example, and data stored in an electronically erasable and programmable read only memory (EEPROM) that is not illustrated can also be referred to, for example.

### Configuration of Storage Unit

The storage unit 200 is constituted by a non-transitory recording medium, such as a digital versatile disk (DVD), a hard disk, or a semiconductor memory and a control unit for the non-transitory recording medium and stores primer pairs each composed of two types of primers that complementarily form a pair with a nucleic acid, an evaluation criteria of non-specific amplification inducibility evaluated between a primer constituting a primer pair and a primer constituting another primer pair, and a solution to a graph coloring problem, and a problem equivalent to the graph coloring problem and a solution thereto that are used when a graph is colored such that vertices adjacent to each other have different colors. In addition, a plurality of primer pairs designed in the design unit 105 are stored. Furthermore, evaluation results of the non-specific amplification inducibility evaluated in the evaluation unit 110 are stored. Furthermore, a graph and a data structure equivalent to the graph generated in the graph generation unit 116 of the assignment unit 115, and a graph and a data structure equivalent to the graph after being colored in the coloring unit 117 are stored.

### Configurations of Display Unit and Operating Unit

The display unit 300 includes the monitor 310 (display device) and is capable of displaying input information, information stored in the storage unit 200, results of processing by the processing unit 100, and so forth. The operating unit 400 includes a keyboard 410 and a mouse 420 each serving as an input device and/or a pointing device. The user can perform operations necessary for performing the design method for dividing primer pairs into reaction containers according to this embodiment by using these devices and via the screen of the monitor 310. The operations that can be performed by the user include, for example, setting of a plurality of target nucleic acids to be amplified and setting of the number of reaction containers. When an extraction step is performed in an assignment step described below, the operations include, for example, the designation of a range of similarity for specifying a primer set and a representative primer pair selected from the primer set.

### Processing in Primer Division-Designing Apparatus

The above-described primer division-designing apparatus 10 is capable of performing a design for dividing primer pairs into reaction containers for dividing primer pairs into reaction containers in accordance with an instruction of the user via the operating unit 400. Design Method for Dividing Primer Pairs into Reaction Containers

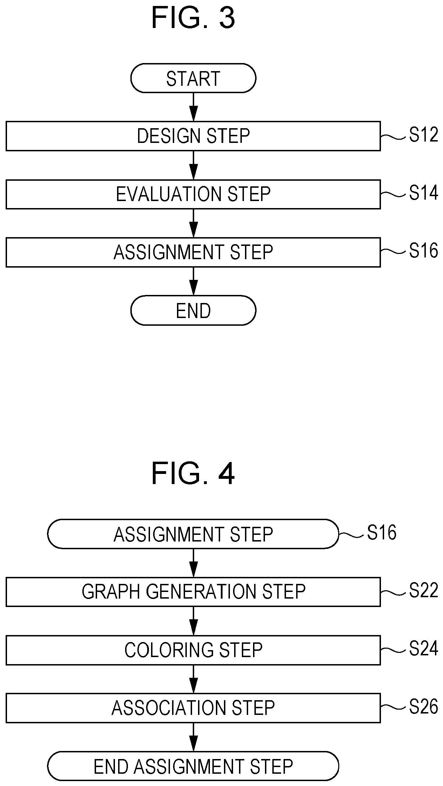

Fig. 3 is a flowchart illustrating a design method for dividing primer pairs into reaction containers according to this embodiment. The design method for dividing primer pairs into reaction containers according to this embodiment is a design method for dividing primer pairs into a plurality of reaction containers to simultaneously amplify a plurality of target nucleic acids and includes a design step of, for each of the plurality of target nucleic acids, designing a primer pair composed of two types of primers that complementarily form a pair to design a plurality of primer pairs, an evaluation step of evaluating non-specific amplification inducibility between a primer constituting a primer pair that forms a pair with one target nucleic acid and a primer constituting a primer pair that forms a pair with another target nucleic acid, and an assignment step of performing an assignment to the plurality of reaction containers on the basis of the non-specific amplification inducibility evaluated in the evaluation step such that primer pairs that include primers having the non-specific amplification inducibility are not present in the same reaction container.

Fig. 4 is a flowchart illustrating steps of the assignment step. The assignment step has a graph generation step of generating a graph having the primer pairs as vertices and non-specific amplification inducibility between primers constituting the primer pairs as an edge or a data structure equivalent to the graph, a coloring step of applying a solution to a graph coloring problem to the graph or applying a problem equivalent to a graph coloring problem and a solution thereto to the data structure equivalent to the graph to color the vertices in a plurality of conceptual colors such that the vertices adjacent to each other with the edge therebetween have different colors, and an association step of associating the plurality of conceptual colors colored in the coloring step with the reaction containers to associate the primer pairs corresponding to the vertices with the reaction containers of the corresponding colors.

The individual steps will be described below.

### Design Step (Step S 12)

The design unit 105 of the designing apparatus 10 performs a design step (step S12). The design step is a step of, for each nucleic acid of a plurality of target nucleic acids, designing a primer pair composed of two types of primers that complementarily form a pair to design a plurality of primer pairs.

First, the selection of a plurality of target nucleic acids is performed. For the plurality of target nucleic acids, genes to be measured are enumerated, and target nucleic acids (base sequences) amplified are selected. For the target nucleic acids, any number of nucleic acids and any target may be selected. In this embodiment, the total number of the target nucleic acids is preferably 50 or more, more preferably 100 or more, and still more preferably 1,000 or more. According to the design method for dividing primer pairs into reaction containers of this embodiment, since primer pairs are divided into reaction container in consideration of non-specific inducibility between primers, even when the number of target nucleic acids is increased, a nucleic acid other than the target nucleic acids can be prevented from being amplified. Therefore, this design method is effective.

The target nucleic acids are not particularly limited and may be, for example, deoxyribonucleic acids (DNAs) and ribonucleic acids (RNAs). The method is particularly effective when there is a high possibility that non-specific reaction is induced. Examples of nucleic acids in which non-specific reaction is highly induced include nucleic acids having a short base sequence, such as non-coding RNAs (ncRNAs) in general, in particular, miRNAs (microRNAs) which are nucleic acids having a short base sequence. The method can be suitably used for these nucleic acids. Nucleic acids having a short base sequence are small RNAs preferably having a number of bases of 200 or less, more preferably 32 or less. The target nucleic acids selected may be not only nucleic acids that are originally short but also nucleic acids that are finely fragmented.

However, if there are strict constraint conditions etc. in the primer design described later, there may be a case where the number of target nucleic acids to be set is large, for example. In this embodiment, any base sequence may be selected as a target nucleic acid regardless of the length of a primer.

Next, for each of the plurality of target nucleic acids, a primer pair that complementarily form a pair is designed. The primer pair is composed of two types of primers that complementarily form a pair from each end of the base sequence of the target nucleic acid. In this embodiment, since a primer pair is designed for each of the plurality of target nucleic acids, a plurality of primer pairs are designed. The design of primer pairs can be set under various conditions depending on the purpose and the like. The type of primer is also not limited. For example, in a case of a miRNA or the like, a stem-loop RT primer may be placed. Evaluation Step (Step S14)

The evaluation unit 110 of the designing apparatus 10 performs an evaluation step (step S14). In the evaluation step, non-specific amplification inducibility is evaluated between a primer constituting a primer pair that forms a pair with one target nucleic acid and a primer constituting a primer pair that forms a pair with another target nucleic acid. The evaluation of non-specific amplification inducibility can be conducted on the basis of the base homology between the primers, for example. The base homology can be calculated by, for example, a local sequence alignment algorithm in which the 3'-end sequence side is fixed. The evaluation can be conducted by checking the number of matches (so called "Matches"), the number of mismatches ("Mismatches"), and insertion/deletion (In/Del) on the basis of the base homology.

However, in this embodiment, the evaluation of non-specific amplification inducibility may be conducted by various methods without being limited to a specific determination method and a specific threshold value. In particular, in the amplification of a miRNA, which is a nucleic acid having a short base sequence, when a stem-loop primer is used for one end, an example of the threshold value of base homology and the number of consecutive matches from the end side are important, and the use of these values is effective for the evaluation of non-specific amplification inducibility. For example, when a complementarity score S is represented by S = m - u - 3d, where m represents the number of matches, u represents the number of mismatches, and d represents the number of insertions/deletions, inducibility of non-specific reaction between the primers is determined from a condition (A): for the stem-loop primer, the number of consecutive matches of bases on the 3'-end-side is 4 or more or the complementarity score S satisfies S > 5, and a condition (B): for an ordinary primer, the complementarity score S satisfies S > 9, and when one of the conditions (A) and (B) is satisfied, the primers are determined to have non-specific amplification inducibility. The complementarity score means the maximum value of a local alignment score under the constraint that the 3'-ends of the primer sequence and another sequence are included. Furthermore, in the comparison between the two sequences in such a case, the number of matches means that bases in the sequences are matched, the mismatch means that bases in the sequences are mismatched, and insertion/deletion means that a base of one of the sequences is deleted or inserted. The "ordinary primer" as used in this embodiment means an ordinary primer relative to a stem-loop primer and refers to a typical linear primer that does not have a loop in a part thereof.

The division of primer pairs into reaction containers is performed on the assumption that combinations of primers that constitute primer pairs to be introduced for target nucleic acids are abundantly present under the condition that any nucleic acid is present. Note that "any nucleic acid" refers to a nucleic acid that is present in a sample, and for example, refers to a nucleic acid known as a human miRNA when amplification is performed in order to measure a specific human miRNA. Accordingly, for example, long nucleic acids, most of which can be excluded by a preliminary pretreatment, may be excluded when the calculation is performed. However, nucleic acids that may not be excluded may be included in the calculation.

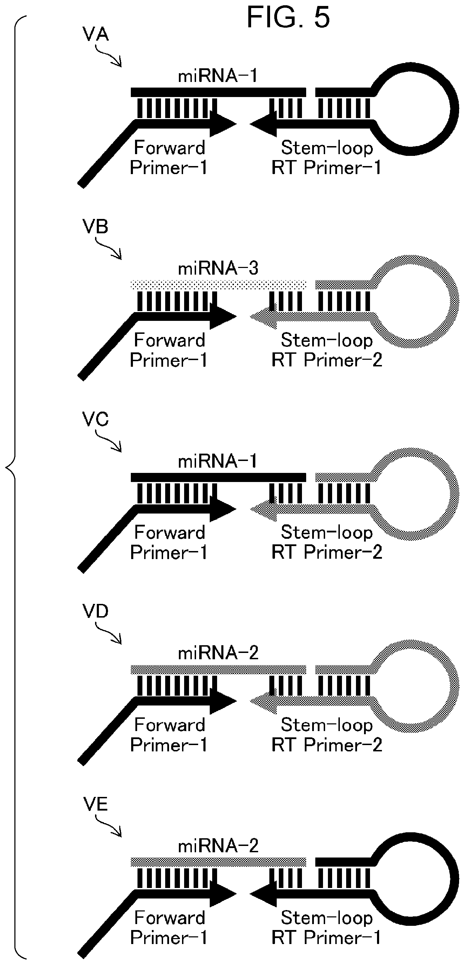

Regarding the non-specific amplification inducibility evaluated in the evaluation step, specifically, the "non-specificity between a pair of introduced primers to which any nucleic acid "is bridged"" is checked. If an introduced primer is bridged to any nucleic acid different from the target nucleic acid to constitute a primer pair, this is regarded as a case where a non-specific amplification is induced. Fig. 5 is a diagram for describing a state where a primer pair is bridged and amplified. In Fig. 5, pattern VA is a drawing illustrating the state of a combination of a normal primer pair. To amplify miRNA-1 as a target nucleic acid, Forward Primer-1 is reacted to the 3'-end-side of miRNA-1, and Stem-loop RT Primer-1 is reacted to the other end side. Note that a description will be made on the assumption that Forward Primer-1 and Stem-loop RT Primer-1 are bridged, so that miRNA-1 is amplified as a target nucleic acid. Similarly, Forward Primer-2 and Stem-loop RT Primer-2 are bridged, so that miRNA-2 is amplified as a target nucleic acid, and Forward Primer-3 and Stem-loop RT Primer-3 are bridged, so that miRNA-3 is amplified as a target nucleic acid.

However, in the amplification of a target nucleic acid, if primers are introduced in the same reaction container without dividing the primers, as illustrated in pattern VB, a pair of Forward Primer-1 and Stem-loop RT Primer-2 may be bridged to miRNA-3, which is different from the target nucleic acid, so that miRNA-3 may be amplified. Alternatively, as illustrated in pattern VC, although Forward Primer-1 is bridged to miRNA-1, which is the target nucleic acid, the other end side may be bridged to Stem-loop RT Primer-2. Alternatively, as illustrated in pattern VD, to the 3'-end-side of miRNA-2, Forward Primer-1, whose target nucleic acid is different, may be bridged, and Stem-loop RT Primer-2 may be bridged to the other end side. In pattern VC, the target nucleic acid miRNA-1 is amplified for Forward Primer-1, and in pattern VD, the target nucleic acid miRNA-2 is amplified for Stem-loop RT Primer-2. However, the amplification is not performed between an appropriate pair of primers. Alternatively, as illustrated in pattern VE, a pair of Forward Primer-1 and Stem-loop RT Primer-1 is bridged to miRNA-2, which is different from the target nucleic acid, so that miRNA-2, which is different from the target nucleic acid, may be amplified. Patterns VB, VC, and VD may be caused by introducing Forward Primer-1 and Stem-loop RT Primer-2 into the same reaction container (tube); therefore, Forward Primer-1 and Stem-loop RT Primer-2 are introduced into separate reaction containers. That is, if Primer pair 1 of Forward Primer-1 and Stem-loop RT Primer-1 and Primer pair 2 of Forward Primer-2 and Stem-loop RT Primer-2 are present in the same reaction container, a nucleic acid different from miRNA-1, which is the target nucleic acid, may be amplified between Forward Primer-1 and Stem-loop RT Primer-2. Thus, such a non-specific amplification can be avoided by introducing Primer pair 1 and Primer pair 2 into separate reaction containers.

As illustrated in pattern VE, Forward Primer-1 and Stem-loop RT Primer-1, which are a primer pair that complementarily form a pair with miRNA-1 serving as a target nucleic acid, may react with miRNA-2 different from the target nucleic acid. In the case of pattern VE, it is difficult to avoid a non-specific amplification; however, it is conceivable that Primer pair 1 and Primer pair 2, which forms a pair with a target nucleic acid miRNA-2, are divided. The case of pattern VE can also be regarded as a division target of this embodiment.

In the evaluation of non-specific amplification inducibility, the target of division may not include all of patterns VB, VC, VD, and VE in Fig. 5 but may be limited to some of the patterns selected from these. In Fig. 5, the description has been made of a pattern of cross-amplification of a miRNA, which is a nucleic acid, and a forward primer and a stem-loop primer. Also in the case where primers are bound to each other to form a dimer, it is necessary to divide primer pairs. In the evaluation of non-specific amplification inducibility, such a binding between primers is also preferably checked.

### Assignment Step (Step S16)

The assignment unit 115 of the designing apparatus 10 performs an assignment step (step S16). The assignment step is a step of assigning primer pairs to a plurality of reaction containers on the basis of the non-specific amplification inducibility evaluated in the evaluation step such that primer pairs including primers having non-specific amplification inducibility are not present in the same reaction container. The assignment step includes a graph generation step (step S22) of generating a graph having primer pairs as vertices, and non-specific amplification inducibility between primers constituting the primer pairs as an edge or a data structure equivalent to the graph, a coloring step (step S24) of applying a solution to a graph coloring problem to the graph or applying a problem equivalent to a graph coloring problem and a solution thereto to the data structure equivalent to the graph to perform coloring with a plurality of conceptual colors such that vertices adjacent to each other with the edge therebetween have different colors, and an association step (step S24) of associating the plurality of individual conceptual colors with the reaction containers to associate the primer pairs with the reaction containers of the corresponding colors to assign the primer pairs to the plurality of reaction containers.

### Graph Generation Step (Step S22)

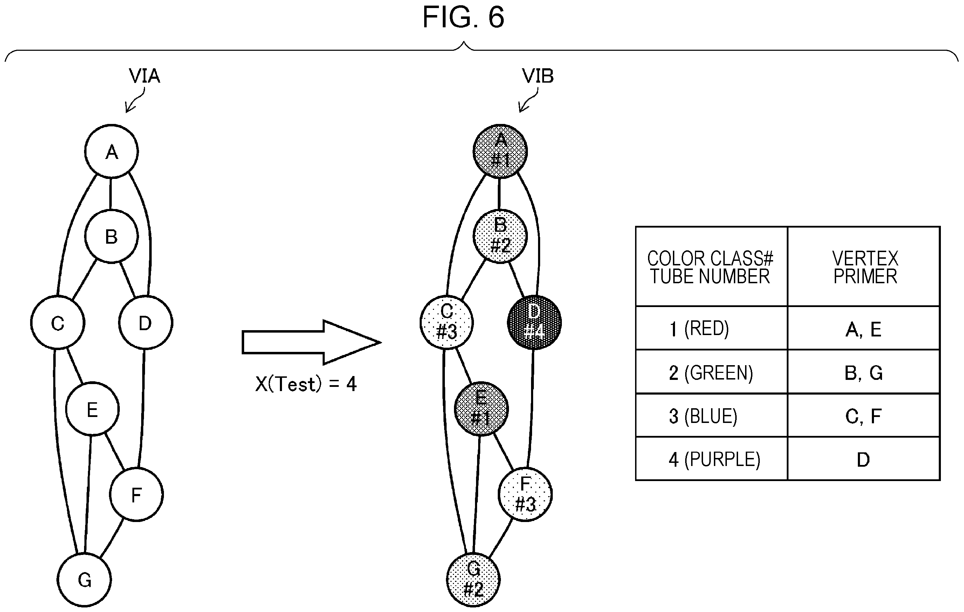

The graph generation unit 116 of the assignment unit 115 performs the graph generation step (step S22). The graph generation step is a step of generating a graph having primer pairs as vertices, and non-specific amplification inducibility between primers constituting primer pairs as an edge. Fig. 6 is a diagram for describing the assignment step, and Graph VIA is a diagram illustrating an example of a primer non-specific graph (graph). Graph VIA is a primer non-specific graph generated for seven primer pairs, in which the primer pairs are indicated by A to G. Each primer pair is denoted by a vertex (node), and primer pairs evaluated in the evaluation step that primers constituting the primer pairs have non-specific amplification inducibility are joined to each other by an edge (side, link). The number of vertices of the graph is equal to the number of primer pairs, that is, the number of target nucleic acids. The number of edges depends on the calculation results in the evaluation step and is equal to the number of cases where primer pairs have non-specific amplification inducibility.

### Coloring Step (Step S24)

The coloring unit 117 of the assignment unit 115 performs the coloring step (step S24). The coloring step is a step of applying a solution to a graph coloring problem to the graph to color the vertices in a plurality of conceptual colors such that vertices adjacent to each other with the edge therebetween have different colors. As described later, the colors individually assigned here correspond to the reaction containers (tubes), and thus the number of colors is preferably small. In addition, when the numbers of vertices colored with respective colors are equal to each other, the number of primer pairs assigned to each reaction container can be equalized. Thus, the coloring step is preferably performed such that the number of vertices colored in each color is equalized. Graph VIB in Fig. 6 is a diagram obtained by coloring the primer non-specific graph illustrated in Graph VIA. As illustrated in Graph VIB, when primer pairs A and E are colored in red, primer pairs B and G are colored in blue, primer pairs C and F are colored in green, and primer pair D is colored in purple, no two adjacent vertices can have the same color.

A problem in which a graph is colored in the minimum number of colors is called a "graph coloring problem", which is well known in graph theory. For example, the Welsh-Powell method is known as a heuristic solution to a graph coloring problem, and the coloring can be performed by using such a method.

Note that although a description has been made herein as a "graph coloring problem" that is the most easily understandable, many of NP-complete problems to which a graph coloring problem belongs have been proved to be equivalent to each other. Thus, such a problem may be modified and solved as another problem, that is, a data structure and an algorithm other than a graph (corresponding to a "data structure equivalent to a graph") may also be applied.

For example, a graph coloring problem can be redefined as an enumeration problem of independent sets and a set covering problem with the independent sets. First, the independent set refers to a set of vertices that are not adjacent to each other on a graph. Furthermore, it is efficient to consider a maximal independent set. This definition makes it clear that in a solution to a graph coloring problem, a set of vertices colored in one certain color constitutes an independent set. However, if there is a vertex that can be colored in overlapping colors between independent sets, the vertex may be assigned to either color class. Thus, conversely, if all of the vertices of the graph can be covered (entire coverage) by vertices included in a plurality of independent sets, the graph coloring problem is considered to be solved.

Furthermore, the complementary set of an independent set is a vertex cover (at least one end point of every edge of the graph is included in the vertex cover). Here, it is known that a vertex cover problem can be modified into a partial sum problem. Accordingly, the graph coloring problem can be modified into problems such as a partial sum problem and a set covering problem, in which a graph does not apparently appear at first sight. While such modified problems and solutions to the problems are considered, primers can be assigned by associating the vertices with reaction containers as in the case of solving the graph coloring problem.

In the above, an equivalence transformation has been described; however, for example, it is possible to perform modification to another problem that satisfies a sufficient condition or modification to another problem for which an approximate solution is obtained. The other problems similar to the graph coloring problem and solutions to these problems are collectively referred to as "a data structure equivalent to a graph and a problem equivalent to a graph coloring problem and a solution thereto". Such a modification can be made in various manners and is effective, for example, in the case of use for various existing good or familiar algorithms, software modules, and the like.

Furthermore, in the coloring step, a search using ZDD may also be performed. Specifically, for example, the above-described coloring problem is divided into "enumeration of maximal independent sets (MIS)" and "enumeration of graph entire coverage by MIS", and ZDD corresponding to each of them can be constructed.

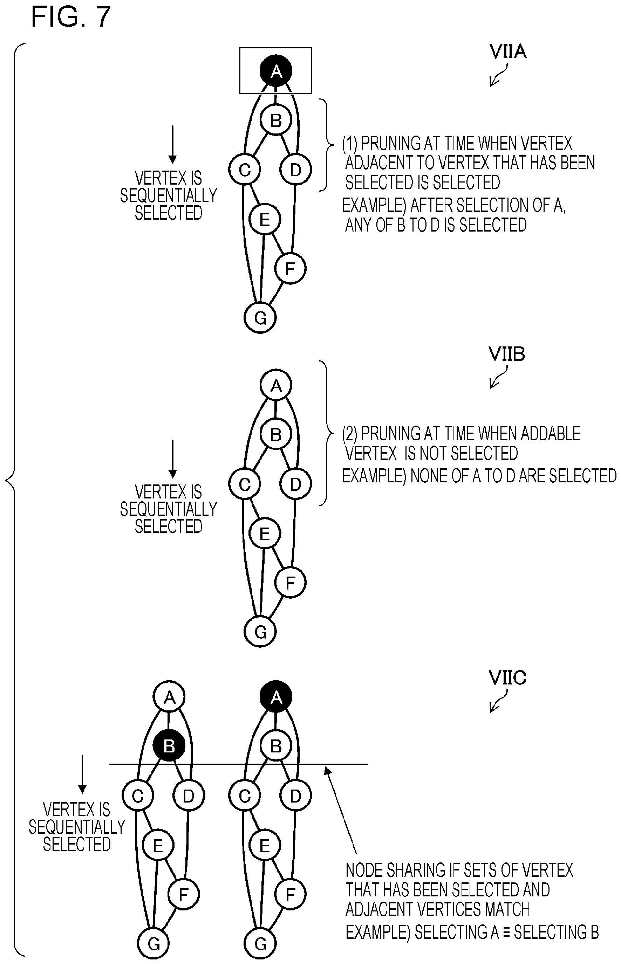

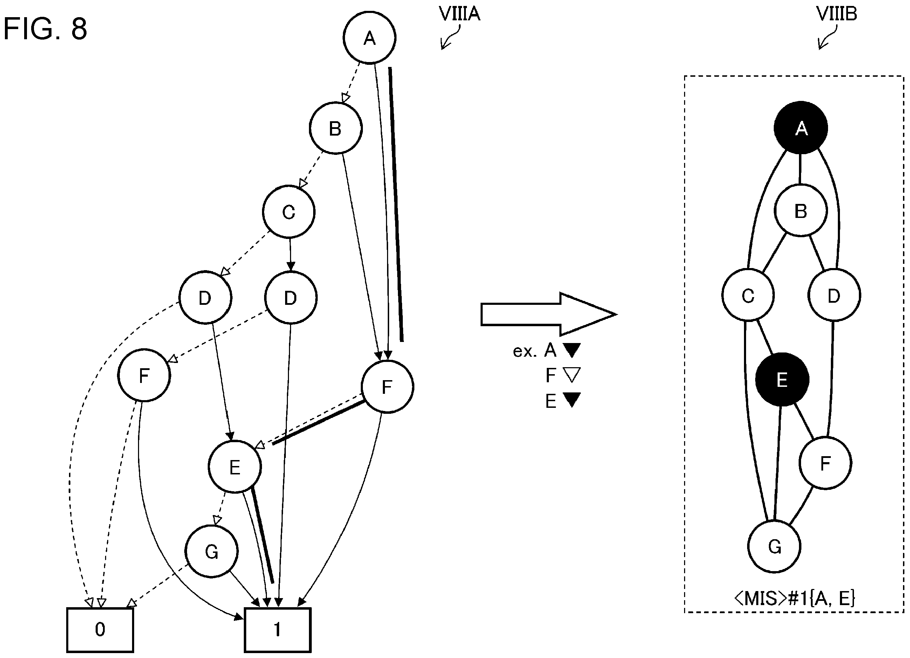

Figs. 7 and 8 are diagrams for describing a procedure for enumerating maximal independent sets of a graph example using ZDD. First, for the primer non-specific graph generated in the graph creation step (step S22), patterns to be discriminated are reduced by "pruning" and "node sharing". The "pruning" refers to a process of reducing patterns to be discriminated, by discriminating whether a selection of a certain vertex is determined to be inappropriate without considering the presence or absence of remaining selections. The "node sharing" refers to a process of reducing patterns to be discriminated by aggregating a group of patterns that are different selection patterns and have the same branch when subsequent selections match each other.

Graph VIIA in Fig. 7 is a diagram for describing a pruning condition (1), and Graph VIIB is a diagram for describing a pruning condition (2). Under the pruning condition (1), "pruning" is performed at the time when a vertex adjacent to a vertex that has been selected is selected. In the coloring step, coloring is performed such that vertices adjacent to each other with an edge therebetween have different colors. Accordingly, at the time of the selection of B, C, or D adjacent to vertex A that has been selected, the combination determined by this selection becomes inappropriate, and thus it is not necessary to consider the subsequent selection. Under the pruning condition (2), "pruning" is performed at the time when an addable (selectable) vertex is not selected. This is because even if vertex A is added to an independent set obtained when none of A to D are selected, the resulting set is still an independent set, and thus the set is not a "maximal" independent set.

Graph VIIC in Fig. 7 is a diagram for describing a node sharing condition. Node sharing occurs at the time when sets of a vertex that has been selected and adjacent vertices match. Specifically, comparing vertex A that has been selected and vertex (B) adjacent to vertex A, vertices adjacent to vertex (A) are vertices B, C, and D, and vertices adjacent to vertex B are vertices A, C, and D. Accordingly, in the case where A is selected and the case where B is selected, the subsequent selections are the same, and thus branches of ZDD are joined together to share the subsequent selection process. As a result, when either vertex A or vertex B is selected, it is not necessary to individually perform the process in a redundant manner. For example, in Fig. 8, selecting A (AV) and not selecting A but selecting B (AVBV) are joined to the same F. Therefore, when either vertex A or vertex B is selected, it is not necessary to individually perform the process in a redundant manner.

When maximal independent sets, that is, all candidates for the color class are enumerated under these conditions, a ZDD representation of the enumeration of maximal independent sets is obtained as illustrated in Graph VIIIA in Fig. 8. With this Graph VIIIA, under the conditions that an arrow ▼ indicates that the vertex is selected, and an arrow ∇ indicates that the vertex is not selected, combinations that can be selected are represented by 1, and combinations that cannot be selected are represented by 0. Specifically, a combination resulting in 1 is a case where any one of vertices adjacent to each other with an edge therebetween is selected, and any one of a vertex which is determined to be selected or not to be selected and a vertex adjacent to the vertex with an edge therebetween is selected. For example, by selecting A, not selecting F, and selecting E in Graph VIIIA, a graph illustrating an extraction example of a maximal independent set can be generated as illustrated in Graph VIIIB.

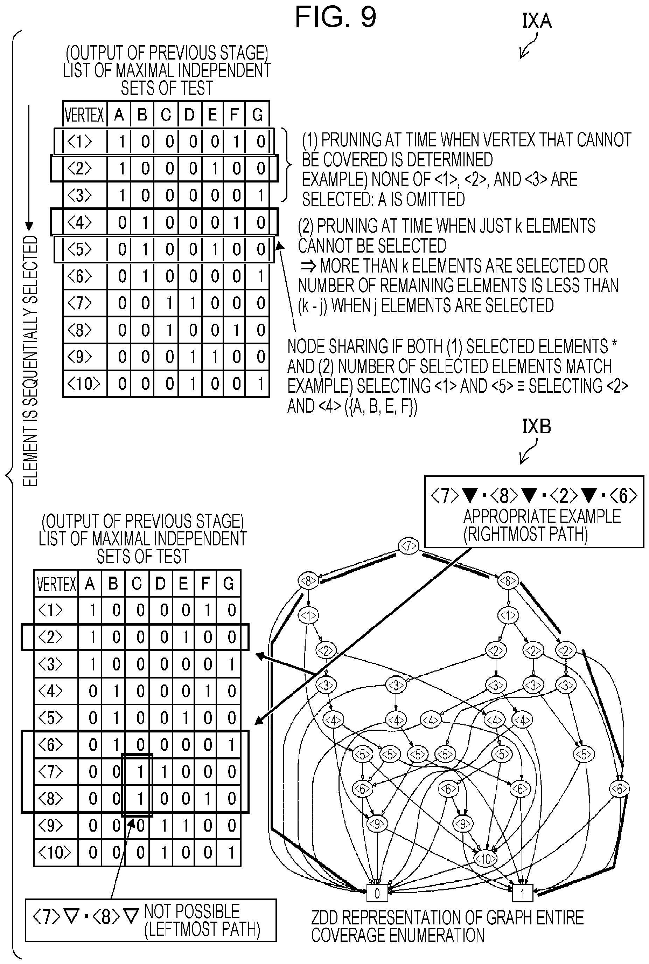

Fig. 9 is a diagram for describing a procedure for enumerating graph entire coverage by MIS using ZDD. Table IXA in Fig. 9 is a list of maximal independent sets of the graph (refer to Graph VIA in Fig. 6) generated in the graph creation step (step S22) and is a table that lists the combinations resulting in 1 in Graph VIIIA in Fig. 8. That is, all the maximal independent sets resulting in 1 are extracted from Graph VIIIA in Fig. 8 in accordance with the procedure described above, and the maximal independent sets are sequentially referred to as elements <1>, <2>, ···, etc. to form a list thereof. In Table IXA, under a pruning condition (1), "pruning" is performed at the time when a vertex that cannot be covered is determined. Specifically, when none of elements <1>, <2>, and <3> are selected, vertex A is not selected, and thus vertex A is not covered, and vertex A is omitted. Therefore, the subsequent selection need not be considered. Under a pruning condition (2), "pruning" is performed at the time when k elements cannot be selected, where k is the number of selections that has been set. For example, when the number of selected elements exceeds k, or when the number of elements to be selected or unselected is less than (k - j) at the time when j elements have been selected, the number of selections cannot be k. Therefore, the subsequent selection need not be considered at this time. Note that k is the number of colors colored in the coloring step.

As for a node sharing condition, when both (1) vertices of selected elements and (2) the number of selected elements match, node sharing occurs. For example, in Table IXA, in the case where elements <1> and <5> are selected and the case where elements <2> and <4> are selected, the selected vertices are {A, B, E, F}, and the number of elected elements is two, which are the same. In this case, if other selected elements are under the same conditions, the coloring results become the same. Accordingly, in each of the combination of elements <1> and <5> and the combination of elements <2> and <4>, the subsequent selections are the same, and thus branches of ZDD are joined together to share the subsequent selection process. As a result, when elements <1> and <5> or elements <2> and <4> are selected, it is not necessary to individually perform the process in a redundant manner.

By performing the "pruning" and "node sharing" described above, patterns to be discriminated can be reduced. By enumerating set covers, that is, final graph coloring results, a ZDD representation of graph entire coverage enumeration, illustrated in Graph IXB can be obtained. A combination with which all of vertices A to G can be selected is determined from Graph IXB. For example, by selecting elements <7>, <8>, <2>, and <6> (▼), all the vertices can be covered. By performing coloring for each element, vertices adjacent to each other can be colored in different colors. In the case where neither <7> nor <8> is selected (V), vertex C is not colored; therefore, this combination is not selected.

### Association Step (Step S26)

The association unit 118 of the assignment unit 115 performs the association step (step S26). The association step is a step of associating the plurality of conceptual colors colored in the coloring step with reaction containers to associate primer pairs corresponding to vertices with reaction containers of the corresponding colors. Primer pairs colored in the same color do not have non-specific amplification inducibility between primers that constitute the primer pairs. Therefore, even when such primer pairs are introduced in the same reaction container, they do not amplify a miRNA different from the target nucleic acid and thus can amplify only the desired target nucleic acid.

Specifically, in the colored primer non-specific graph (refer to Graph VIB) illustrated in Fig. 6, red is defined as tube 1, and primers A and E are assigned thereto; green is defined as tube 2, and primers B and G are assigned thereto; blue is defined as tube 3, and primers C and F are assigned thereto; and purple is defined as tube 4, and primer D is assigned thereto. As a matter of course, any association between the number and the color may be used.

In the case where a multiplex PCR operation is assumed, in general, it is preferred that the number of tubes be small and that the numbers of primers included in the tubes be equalized. Note that an assigned color may be optionally further divided into two or more. However, conversely, combining colors together is not preferred because there is a possibility that a combination of primer pairs having non-specific amplification inducibility is generated.

Note that the division design as used in this embodiment refers to a configuration method for assigning primers to reaction containers, and the preparation of physical primers is not necessary essential. For example, it is also possible to constitute a service in which information about target nucleic acids to be amplified and the like is input from the web, and an assignment to reaction containers (a list in which primer pairs to be divided into reaction containers are divided into groups) is returned.

A tube set may also be provided as a set of reaction containers that include primer pairs divided into reaction containers (tube set). When a target nucleic acid to be amplified is determined, a primer pair that forms a pair is also determined. Accordingly, primer pairs that do not have non-specific amplification inducibility between the primer pairs can be assigned to a tube to provide a tube set. In the evaluation of the non-specific amplification inducibility, in the case where one side of a primer pair is a stem-loop primer, it is determined to have non-specific amplification inducibility when, for example, the following conditions are satisfied: (A) for inducibility of non-specific reaction of the stem-loop primer, the number of consecutive matches of bases on the 3'-end-side is 4 or more or the number of mismatches is less than two, and (B) the number of matches of an ordinary primer is equal to or more than the sum of the number of mismatches and a value three times the number of insertions/deletions. Therefore, primer pairs are divided into a tube set such that such primer pairs are not included in the same reaction container.

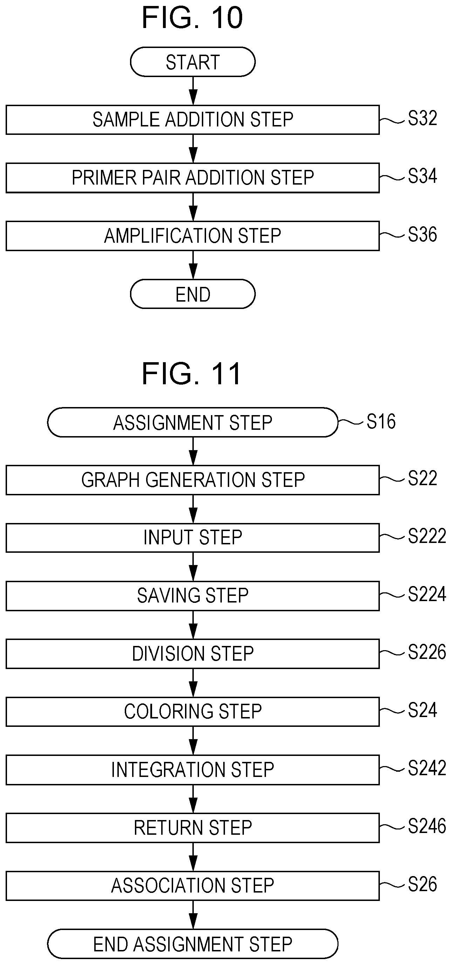

Next, a method for amplifying target nucleic acids will be described. Fig. 10 is a flowchart illustrating a method for amplifying target nucleic acids. The method for amplifying target nucleic acids has a sample addition step (step S32) of adding a sample including a plurality of target nucleic acids to a plurality of reaction containers, a primer pair addition step (step S32) of, on the basis of the above-described design method for dividing primer pairs into reaction containers, separately adding the primer pairs to the corresponding reaction containers, and a step of amplifying the target nucleic acids in the reaction containers.

### Sample Addition Step (Step S32)

When target nucleic acids are actually amplified, first, a sample is added to a plurality of reaction containers. The addition of the sample may be performed by an ordinary method. Primer Pair Addition Step (Step S34)

The primer pair addition step is a step of adding primer pairs assigned to the plurality of reaction containers in the assignment step (step S16) to the corresponding reaction containers.

### Amplification Step (Step S36)

The amplification step is a step of amplifying the target nucleic acids using the reaction containers to which the sample and the primer pairs are added in the sample addition step (step S32) and the primer pair addition step (step S34). The amplification step may be performed by an ordinary method with reference to various literatures.

After the amplification step, information about the amplified nucleic acids can be read to acquire information about the cells. When the information about the nucleic acids is read by next-generation sequencing (NGS), the information can be read as a unified sample in which reaction products after multiplex PCR reaction are mixed.

### Other Embodiments

In the above embodiment, a case where the number of vertex primers is seven has been described. In reality, however, it is necessary to divide a very large number of primer pairs. In order to improve the efficiency of calculation, various methods are employed. The calculation can be performed in embodiments described in the following modifications.

### First Modification

A first modification differs from the above embodiment in that, in the assignment step (step S16), after the graph generation step (step S22), among vertices of the generated primer non-specific graph, vertices whose number (k - 1 or less) is smaller than the number (k) of colors colored in the coloring step are recursively saved.

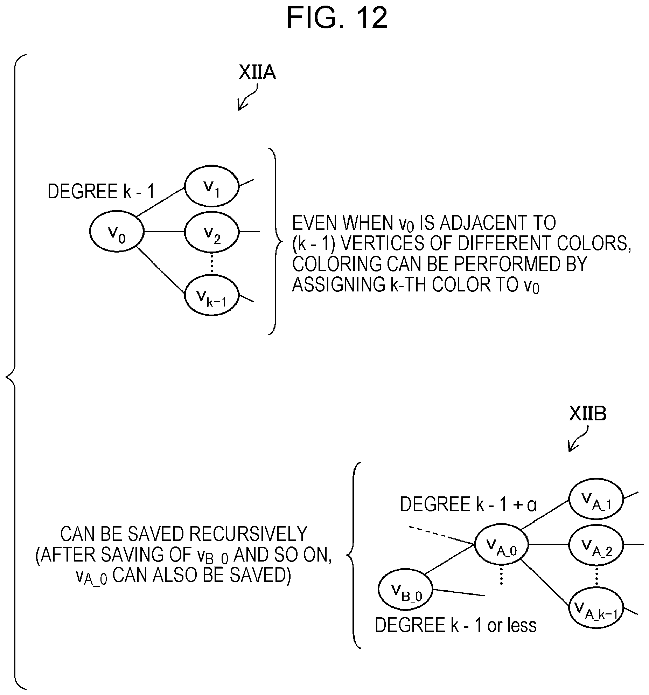

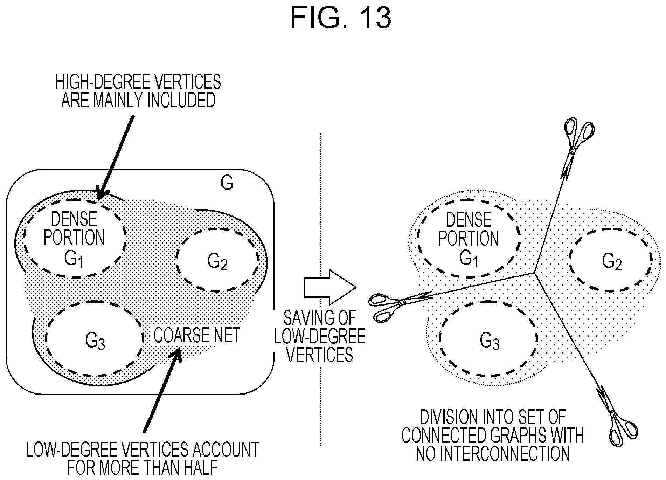

Fig. 11 is a flowchart illustrating steps of an assignment step of the first modification. Fig. 12 is an image diagram of a saving step of the first modification, and Fig. 13 is an image diagram of a division step.

In vertex saving, specifically, an input step (step S222) of inputting k, if k colors (= the number k of reaction containers) is allowable, is performed. Next, a saving step (step S224) of recursively saving a vertex with a degree of (k - 1) or less is performed. Herein, the degree refers to the number of edges connected to the vertex.

As illustrated in Graph XIIA in Fig. 12, for vertex V₀ with a degree of (k - 1), when (k - 1) vertices adjacent to vertex V₀ are colored in different colors and vertex V₀ is colored in the k-th color, vertices adjacent to each other can be colored in different colors. Accordingly, by saving the vertices with a degree of (k - 1) or less and coloring the saved graph, the coloring step (step S24) can be simplified. As illustrated in Graph XIIB, vertex V_{A_0} with a degree of (k - 1 + α) [where α is the number of edges adjacent to vertices with a degree of (k - 1) or less] is adjacent to vertex V_{B_0} with a degree of (k - 1) or less with an edge therebetween; however, vertex V_{B_0} can be saved because V_{B_0} has a degree of (k - 1) or less. Accordingly, if the degree of vertex V_{A_0} becomes (k - 1) or less as a result of saving such a vertex that can be saved, the vertex can also be saved.

The allowable numerical value k may be set to any number. In the case where a division multiplex PCR operation is assumed, for example, if an operation using an 8-channel pipette is assumed, k is preferably 8, and if an operation using a 12-channel pipette is assumed, k is preferably 12. The numerical value k may also be determined in consideration of the reagent cost due to division. That is, k may be set on the basis of various criteria such as workability and the cost.

By performing the saving step (step S224), it is possible to increase the possibility that each of the saved graphs can be divided into a plurality of graphs (connected graphs). As illustrated in Fig. 13, when the divided graphs are separated into dense portions that mainly include high-degree vertices and a coarse net region where low-degree vertices account for more than half, a division step (step S226) of saving low-degree vertices (dividing in a portion of low-degree vertices) is performed. By performing the dividing step, the coloring step (step S24) can be further simplified.

After the division step (step S226) is performed, for each of the connected graphs, coloring the graph is performed (coloring step). The coloring of the graph can be performed by the method described above. By performing the saving step (step S224) and the division step (step S226) and performing the coloring step (step S24) for each connected graph using the ZDD described above, the exhaustive search can be efficiently performed, and necessity and sufficiency of the number of necessary reaction containers (number of tubes) can be ensured.

After the coloring step (step S24) is performed, an integration step (step S242) of integrating the connected graphs saved in the division step (step S226) to generate the graph is performed.

After the integration step (step S242) is performed, a return step (step S246) of returning the vertices with a degree of (k - 1) or less saved in the saving step (step S224) is performed. Since the saved vertices have a degree of (k - 1) or less, by coloring the saved vertices in colors different from those of adjacent vertices adjacent to the saved vertices, the saved vertices can be colored in colors different from the colors of the adjacent vertices. Furthermore, vertices are not adjacent to each other between the graphs saved in the saving step (step S224) and between the connected graphs saved in the division step (step S226); therefore, the coloring results may be simply integrated, or the vertices can be colored again in each of the graphs using the maximum number of colors to color the entire graph.

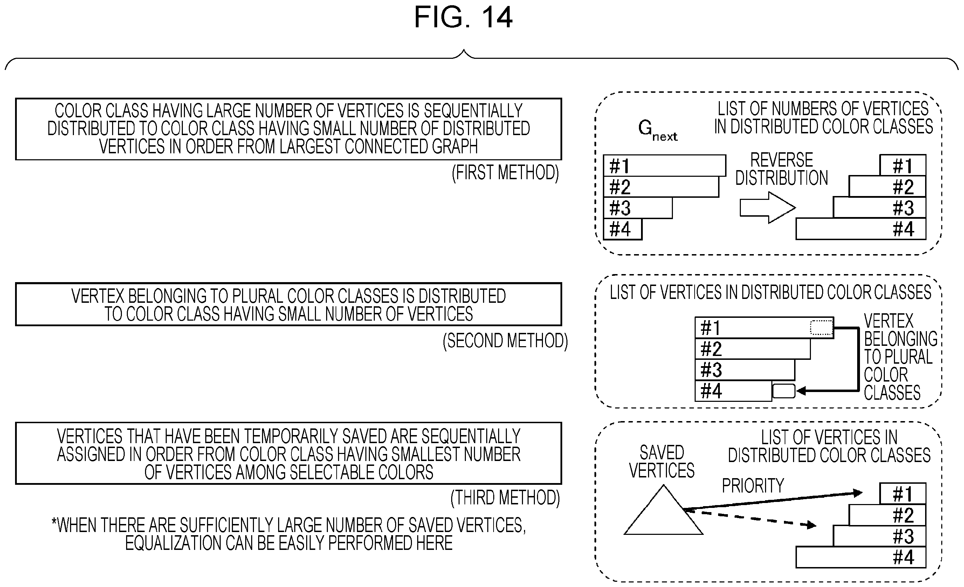

Fig. 14 is an image diagram of the integration step and the return step of the first modification. When graphs where vertices have been saved or divided graphs are returned, coloring is preferably performed such that the number of vertices belonging to the class of each color, that is, the number of primer pairs to be divided into each reaction container is equalized. Specifically, such equalization can be achieved by adjusting, for example, integration of the graph coloring results, distribution of overlapping vertices of maximal independent sets, and assignment of the vertex return.

In a first method, when connected graphs are returned, a color class having a large number of vertices in a connected graph is distributed to a color class having a small number of vertices in an original colored graph. In a second method, if a vertex can be distributed to any of a plurality of colors, the vertex is distributed to a color class having a small number of vertices. In a third method, saved vertices are sequentially distributed in the order from the color class having the smallest number of vertices among the selectable colors. When there are a large number of saved vertices, the numbers of primer pairs in the reaction containers can be equalized by the third method.

After the return step (step S246) is performed, the association step (step S26) is performed to associate the plurality of colors colored in the coloring step with the reaction containers, thereby associating the primer pairs corresponding to the vertices with the reaction containers of the corresponding colors.

In the first modification, graph coloring can be efficiently performed by the above method. In addition, since the numbers of primers in the reaction containers can also equalized, the numbers of target nucleic acids to be amplified can also be equalized in the respective reaction containers.

Since graph coloring problems are difficult problems, the reduction in the graph scale is very important. By reducing the number of vertices adjacent to each vertex on the basis of appropriate vertex saving and graph division, the efficiency of the graph coloring search can be significantly improved. In addition, if the coloring result is finally k or more, the number of colors after vertex saving satisfies necessary and sufficient conditions for the original number of colors. The same also applies to the graph division.

In Fig. 11, the description has been made of a method in which both the saving step (step S224) and the division step (step S226) are performed. Alternatively, for example, when the number of vertices is sufficiently small, the assignment step can be performed by performing only one of the saving step (step S224) and the division step (step S226). In Fig. 11, when the saving step (step S224) is performed, the return step (step S246) is performed. When the division step (step S226) is performed, the integration step (step S242) is performed.

### Second Modification

In a second modification, a representative primer pair is extracted in the graph generation step (step S22) from a primer set consisting of primer pairs having high similarity, and graph coloring is performed by using vertices corresponding to this primer pair to improve the efficiency of graph coloring.

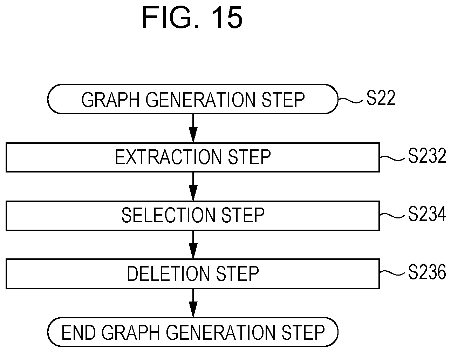

Fig. 15 is a flowchart illustrating steps of a graph generation step of the second modification. The graph generation step of the second modification has an extraction step (step S232) of specifying and extracting, from the primer pairs, a primer set consisting of primer pairs having high similarity, a selection step (step S234) of selecting one or more representative primer pairs from the primer group, and a deletion step (step S236) of excluding the target nucleic acids that form pairs with primers that have not been selected as the representative primers in the selection step among the primer pairs included in the primer set and deleting, in the graph, vertices of the primer pairs corresponding to the excluded target nucleic acids and an edge adjacent to the vertices. Note that the "primer set consisting of primer pairs having high similarity" refers to a set of primer pairs each having a plurality of primer pairs having non-specific amplification inducibility.

In the graph generation step of the second modification, the extraction step (step S232) is first performed. Primer pairs included in the primer set having high similarity each have high non-specific amplification inducibility to a plurality of primer pairs. Therefore, in the generated non-specific graph, a large number of edges are generated from one vertex, and the edges are each connected to a vertex. Accordingly, a plurality of colors are necessary in coloring the vertices. As a result, excessive tubes are required in the subsequent coloring step (step S24).

For example, when 16 target nucleic acids X1, X2, ···, X16 form a clique (a subgraph that forms a complete graph) in a graph, vertex saving as described in the first modification cannot be carried out in the case of k < 16, and thus it is determined that the graph coloring requires 16 or more colors.

On the other hand, if the target nucleic acids X1, X2, ···, X16 are similar to each other, it is originally difficult to discriminate and count these nucleic acids. Accordingly, any one or only a small number of the target nucleic acids X1, X2, ···, X16 are selected as a measurement target (selection step (step A234)), and the others are excluded from the measurement target, that is, vertices of primer pairs corresponding to the target nucleic acids that have been excluded from the measurement target are deleted from the non-specific graph (deletion step (step S236)). As a result, it is possible to reduce the number of vertices of primers connected with edges to primers corresponding to the target nucleic acids to be measured. Accordingly, the number of colors used for graph coloring can be reduced to reduce the number of tube divisions.

The degree of the revealed clique is a candidate for the degree k to be subj ected to vertex saving described in the first modification. The degree of the clique is the lower limit of the number of colors, and satisfies the necessary and sufficient conditions even if the degree of the clique is associated with k. On the other hand, when a complete graph or a dense graph equivalent to the complete graph is obtained by performing vertex saving at an appropriate k and further dividing a graph, a candidate for the representative of the target nucleic acid for representing the gene is provided.

For the sake of simplifying the description, the description has been made with a clique. Alternatively, also in the case where a dense subgraph equivalent to a clique can be extracted, a similar effect can be obtained by selecting any one type or a small number of types of nucleic acids from the dense subgraph as a measurement target. For example, the vertex saving process at a degree of (k - 1) or less and the graph division described in this embodiment are one of methods for extracting a dense subgraph.

In the second modification, graph coloring can be efficiently performed by the above method. In addition, any one or more target nucleic acids among the similar target nucleic acids are amplified and tested, and the test results can be inferred by testing the target nucleic acids similar to the excluded nucleic acids.

### Examples

The present invention will be described more specifically below with reference to Examples of the present invention.

### Example 1 (Dry Examination)

A primer design of a total of 2,656 types of human miRNAs is aimed. Of these, isolated vertices that were independent from other primers (vertices corresponding to primers that did not exhibit non-specific inducibility with any primer) were removed, and a graph having a number of vertices of 1,178 (maximum degree: 30) was then set as a coloring target. At this time, the largest connected graph had a number of vertices of 777. In order to confirm the effect of the invention, first, the check specific to a stem-loop primer is omitted.

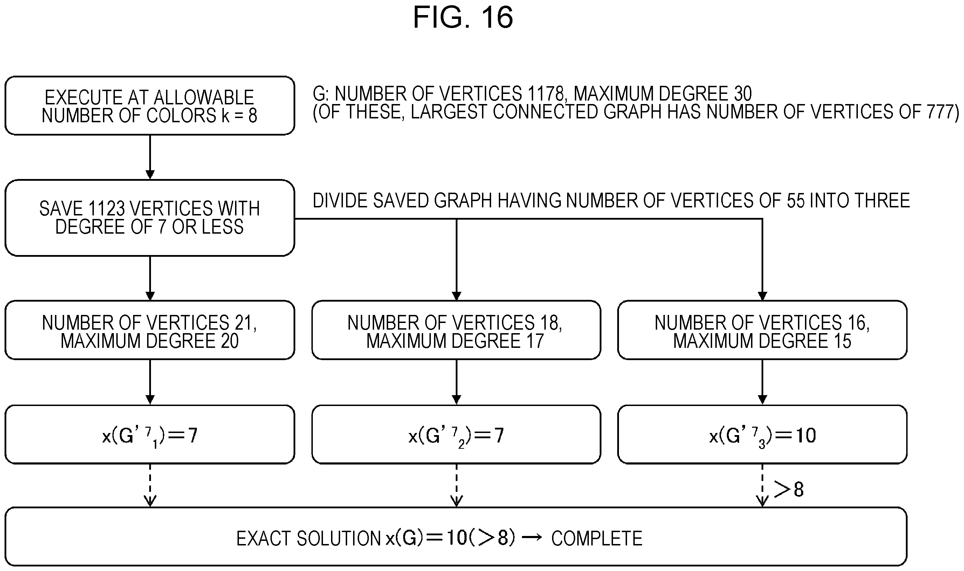

Fig. 16 is a flowchart for describing a design method for dividing primer pairs into reaction containers in Example 1. In Example 1, the execution is carried out at an allowable coefficient k of 8. First, vertices with a degree k of 7 or less are saved. The number of vertices with a degree k of 7 or less was 1,123. By saving vertices with a degree k of 7 or less, the graph could be divided into three. The graphs were a first graph with a number of vertices of 21 and a maximum degree of 20, a second graph with a number of vertices of 18 and a maximum degree of 17, and a third graph with a number of vertices of 16 and a maximum degree of 15.

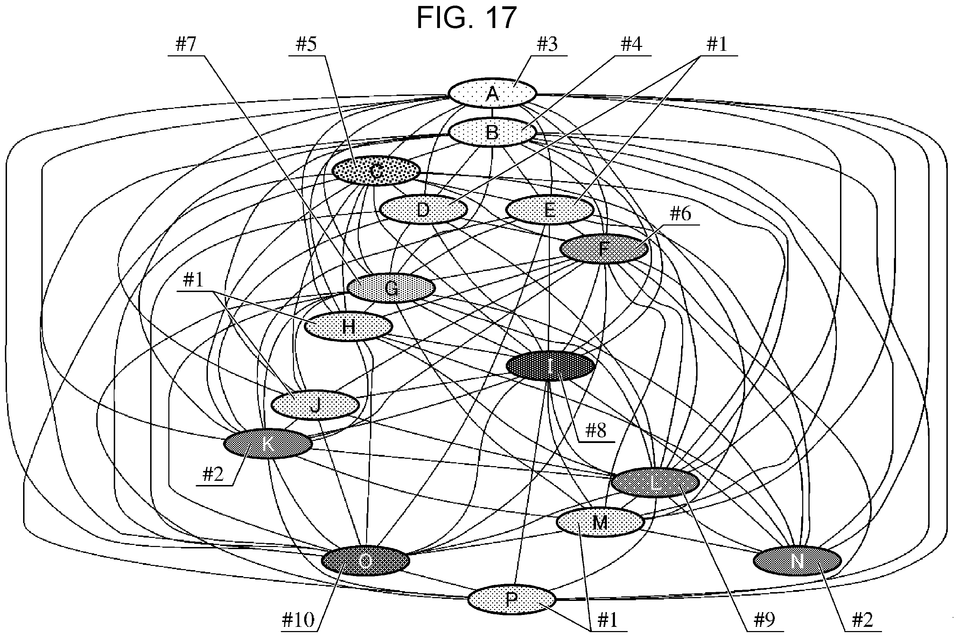

Next, for each of the graphs, graph coloring was performed by ZDD. According to the results, the first graph could be colored with 7 colors (χ(G'⁷₁ = 7)), the second graph could be colored with 7 colors (χ(G'⁷₂ = 7)), and the third graph could be colored with 10 colors (χ(G'⁷₃ = 10)). Fig. 17 illustrates an example of a subgraph coloring of the third graph. The third graph is colored in 10 colors of [#1] D, E, H, J, M, and P (red), [#2] K and N (orange), [#3] A (yellow-green), [#4] B (green), [#5] C (yellow), [#6] F (blue), [#7] G (navy blue), [#8] I (indigo blue), [#9] L (purple), and [#10] O (pink). Although not performed in this Example, a group of primers of the third graph may be represented by any of the primers to thereby reduce the number of colors.

Next, the divided graphs are returned. The number vertices of each color class (number of primers divided into each reaction container) after return is shown in Table 1 below. When the three divided graphs are returned, a color class having a large number of vertices is distributed to a color class having a small number of vertices that have been distributed. The vertices that have been saved are sequentially assigned in the order from the color class having the smallest number of vertices among the selectable colors. In Example 1, the colors (primers) can be evenly assigned to respective colors (respective reaction containers) by assigning the colors in this manner. Table 1 shows, as a reference example, Comparative Example in which the distribution was performed by an existing heuristic method. As shown in Table 1, it was confirmed that the number of vertices could be equalized in the method for Example 1 compared with Comparative Example.

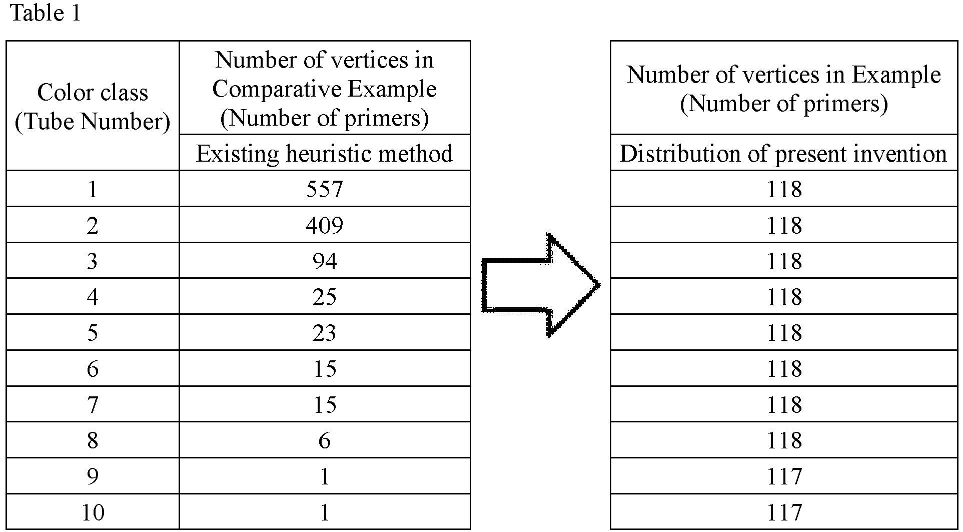

### Example 2 (Dry and Wet Examinations)

For hsa-let-7a-5p that is widely expressed in human, 15 types of forward primers and 54 types of stem-loop primers in which some of bases of the reverse complementary sequence were replaced were prepared. The ΔCt value was checked by qPCR, and a threshold value for designing primers that exhibit non-specific amplification inducibility was examined. In the case of [ΔCt < 8], it was determined that non-specific amplification occurred.

The forward primers were designed in accordance with WO2016/159132A. With regard to the stem-loop primers, on the basis of the sequence characteristics of 12 types of primers that exhibited non-specific amplification, the threshold value for designing primers that exhibit non-specific amplification was set such that the number of consecutive matches on the 3'-end-side was 4 or more or the number of mismatches was less than 2. Non-specific amplification of primers under 54 conditions was determined using the set values. According to the results, 12 cases with problems could be determined to have non-specific amplification. The results are shown in Table 2. Note that the "determination" represents a result determined in advance by the parameters disclosed in the present invention, and the "result" represents a result generated by actual amplification. When non-specific amplification actually occurred, of course, the results showed that non-specific amplification occurred; however, all of these results could be determined in advance that non-specific amplification occurs. Thus, the parameters disclosed in the present invention could be confirmed to function effectively.

**Table 2**

| Non-specific amplification | | Results | |
|---|---|---|---|
| | | Occur | Not occur |
| Determination | Occur | 12 | 9 |
| | Not occur | 0 | 33 |

Subsequently, multiplex PCR was performed using 177 types of miRNAs [Ferguson, Scott W., et al., 2018] that are characteristically highly expressed in mesenchymal stem cells (MSC) to evaluate the effectiveness of the tube division design method.

As comparison targets, level 1 (no division: 188 types of NG pairs determined to cause non-specific amplification), level 2 (name-order division rearranged in the name order: 85 types of NG pairs determined to cause non-specific amplification), and level 3 (name-order redivision further rearranged in the name order at random: 29 types of NG pairs determined to cause non-specific amplification) were prepared, and measurement accuracy was compared.

Compared with the individual qPCR measured value (indicator of measurement accuracy), the measurement accuracy (R² compared with qPCR) decreased depending on the number of NG pairs, and thus it could be confirmed that the division by the design method for dividing primer pairs into reaction containers according to the present invention is effective to improve the measurement accuracy of miRNA.

**Table 3**

| | Example 2 | Level 3 (Name-order redivision) | Level 2 (Name-order division) | Level 1 (No division) |
|---|---|---|---|---|
| R² compared with qPCR | 0.77 | 0.71 | 0.67 | 0.64 |
| Number of NG pairs | 0 | 29 | 85 | 188 |

### Reference Signs List

- 10: primer division-designing apparatus
- 100: processing unit
- 105: design unit
- 110: evaluation unit
- 115: assignment unit
- 116: graph generation unit
- 117: coloring unit
- 118: association unit
- 120: output unit
- 125: display control unit
- 130: CPU
- 135: ROM
- 140: RAM
- 200: storage unit
- 300: display unit
- 310: monitor
- 400: operating unit
- 410: keyboard
- 420: mouse
- 500: external server
- 510: external database
- NW: network

## Claims

1. A design method for dividing primer pairs into a plurality of reaction containers to simultaneously amplify a plurality of target nucleic acids, the design method comprising:
a design step of, for each of the plurality of target nucleic acids, designing a primer pair composed of two types of primers that complementarily form a pair to design a plurality of primer pairs;
an evaluation step of evaluating non-specific amplification inducibility between a primer constituting a primer pair that forms a pair with one target nucleic acid and a primer constituting a primer pair that forms a pair with another target nucleic acid; and
an assignment step of performing an assignment to the plurality of reaction containers, based on the non-specific amplification inducibility evaluated in the evaluation step, such that primer pairs including primers having the non-specific amplification inducibility are not present in the same reaction container,
wherein the assignment step has
a graph generation step of generating a graph having the primer pairs as vertices and non-specific amplification inducibility between primers constituting the primer pairs as an edge or a data structure equivalent to the graph,
a coloring step of applying a solution to a graph coloring problem to the graph or applying a problem equivalent to a graph coloring problem and a solution thereto to the data structure equivalent to the graph to color the vertices in a plurality of conceptual colors such that the vertices adjacent to each other with the edge therebetween have different colors, and
an association step of associating the plurality of conceptual colors colored in the coloring step with the reaction containers to associate the primer pairs corresponding to the vertices with the reaction containers of the corresponding colors.

2. The design method for dividing primer pairs into reaction containers according to claim 1, wherein the graph generation step has
an extraction step of specifying and extracting, from the primer pairs, a primer set consisting of primer pairs having high similarity,
a selection step of selecting one or more representative primer pairs from the primer set, and
a deletion step of excluding the target nucleic acids that form pairs with primer pairs that have not been selected as the representative primer pairs in the selection step among the primer pairs included in the primer set and deleting, in the graph, vertices of the primer pairs corresponding to the excluded target nucleic acids and an edge in contact with the vertices.

3. The design method for dividing primer pairs into reaction containers according to claim 1 or 2, wherein in the coloring step, the number of vertices colored in each color is equalized.

4. The design method for dividing primer pairs into reaction containers according to any one of claims 1 to 3,
wherein the assignment step has, after the graph generation step,
an input step of inputting the number k of the plurality of reaction containers, and
a saving step of saving the vertices with a number of the edges of (k - 1) or less from the graph, and
the assignment step has, after the saving step followed by the coloring step,
a return step of returning the saved vertices with a number of the edges of (k - 1) or less.

5. The design method for dividing primer pairs into reaction containers according to claim 4, wherein in the return step, return is performed such that the number of the primer pairs divided into each of the reaction containers is equalized.

6. The design method for dividing primer pairs into reaction containers according to claim 4, wherein in the return step, return is performed such that the number of colors of each of the plurality of conceptual colors colored in the coloring step is equalized.

7. The design method for dividing primer pairs into reaction containers according to any one of claims 1 to 6,
wherein the assignment step has, after the graph generation step,
a division step of dividing the graph into connected graphs that are independent from each other, and
an integration step of, after the coloring step being performed for the connected graphs, integrating the connected graphs to generate the graph.

8. The design method for dividing primer pairs into reaction containers according to any one of claims 1 to 7,
wherein the target nucleic acids are each a small RNA having a number of bases of 200 or less,
one side of each of the primer pairs is a stem-loop primer, and
in the evaluation step, when a complementarity score S is represented by S = m - u - 3d, where m represents the number of matches, u represents the number of mismatches, and d represents the number of insertions/deletions, inducibility of non-specific reaction between the primers is determined from: (A) for the stem-loop primer, the number of consecutive matches of bases on a 3'-end-side is 4 or more or the complementarity score S satisfies S > 5 and (B) for an ordinary primer, the complementarity score S satisfies S > 9, and when one of (A) and (B) is satisfied, the primers are determined to have non-specific amplification inducibility.

9. The design method for dividing primer pairs into reaction containers according to any one of claims 1 to 8, wherein the target nucleic acids each have a number of bases of 32 or less.

10. The design method for dividing primer pairs into reaction containers according to any one of claims 1 to 9,
wherein the coloring step employs a method based on a graph coloring problem, and
coloring results are searched using ZDD.

11. The design method for dividing primer pairs into reaction containers according to claim 10,
wherein the coloring step employs a method based on a graph coloring problem, and
the coloring results are searched by, using ZDD, enumerating maximal independent sets on the graph and determining a combination that covers vertices of the graph with some or all of the enumerated maximal independent sets.

12. A method for amplifying target nucleic acids, the method comprising:
a step of adding a sample including a plurality of target nucleic acids to a plurality of reaction containers;
a step of adding, based on the design method for dividing primer pairs into reaction containers according to any one of claims 1 to 11, the primer pairs to the corresponding reaction containers; and
a step of amplifying the target nucleic acids in the reaction containers.

13. A tube set comprising:
a plurality of tubes for simultaneously amplifying a plurality of target nucleic acids having a number of bases of 32 or less, wherein
each tube of the plurality of tubes includes, for each of the plurality of target nucleic acids, at least one primer pair composed of two types of primers that complementarily form a pair,
one side of the primer pair is a stem-loop primer, and
when two or more of the primer pairs are included in one of the tubes and a complementarity score S is represented by S = m - u - 3d, where m represents the number of matches, u represents the number of mismatches, and d represents the number of insertions/deletions, inducibility of non-specific reaction between the primers in the tube is determined from: (A) for the stem-loop primer, the number of consecutive matches of bases on a 3'-end-side is 4 or more or the complementarity score S satisfies S > 5 and (B) for an ordinary primer, the complementarity score S satisfies S > 9, and the tube set includes no primer pair that satisfies one of (A) and (B).

14. The tube set according to claim 13, wherein a total number of the plurality of target nucleic acids is 50 or more.

15. The tube set according to claim 14, wherein the total number of the plurality of target nucleic acids is 100 or more.

16. A list of primer pairs divided into a plurality of groups for simultaneously amplifying a plurality of target nucleic acids having a number of bases of 32 or less, wherein
each group of the plurality of groups includes, for each of the plurality of target nucleic acids, at least one primer pair composed of two types of primers that complementarily form a pair,
one side of the primer pair is a stem-loop primer, and
when two or more of the primer pairs are included in one of the groups and a complementarity score S is represented by S = m - u - 3d, where m represents the number of matches, u represents the number of mismatches, and d represents the number of insertions/deletions, inducibility of non-specific reaction between the primers in the group is determined from: (A) for the stem-loop primer, the number of consecutive matches of bases on a 3'-end-side is 4 or more or the complementarity score S satisfies S > 5 and (B) for an ordinary primer, the complementarity score S satisfies S > 9, and the list of primer pairs includes no primer pair that satisfies one of (A) and (B).

17. The list of primer pairs according to claim 16, wherein a total number of the plurality of target nucleic acids is 50 or more.

18. The list of primer pairs according to claim 17, wherein the total number of the plurality of target nucleic acids is 100 or more.

19. A program for dividing primer pairs into a plurality of reaction containers to simultaneously amplify a plurality of target nucleic acids that are each a small RNA having a number of bases of 200 or less, the program comprising:
a step of, for each of the plurality of target nucleic acids, designing a primer pair composed of two types of primers which complementarily form a pair and one of which is a stem-loop primer to design a plurality of primer pairs;
a step of evaluating non-specific amplification inducibility between a primer constituting a primer pair that forms a pair with one target nucleic acid and a primer constituting a primer pair that forms a pair with another target nucleic acid; and
a step of performing an assignment to the plurality of reaction containers, based on the non-specific amplification inducibility evaluated in the step of evaluating the non-specific amplification inducibility, such that primer pairs including primers having the non-specific amplification inducibility are not present in the same reaction container,
wherein the step of performing an assignment has
a step of generating a graph having the primer pairs as vertices and non-specific amplification inducibility between primers constituting the primer pairs as an edge or a data structure equivalent to the graph,
a coloring step of applying a solution to a graph coloring problem to the graph or applying a problem equivalent to a graph coloring problem and a solution thereto to the data structure equivalent to the graph to color the vertices in a plurality of conceptual colors such that the vertices adjacent to each other with the edge therebetween have different colors, and
a step of associating the plurality of conceptual colors colored in the coloring step with the reaction containers to associate the primer pairs corresponding to the vertices with the reaction containers of the corresponding colors, and
in the step of evaluation, when a complementarity score S is represented by S = m - u - 3d, where m represents the number of matches, u represents the number of mismatches, and d represents the number of insertions/deletions, inducibility of non-specific reaction between the primers is determined from: (A) for the stem-loop primer, the number of consecutive matches of bases on a 3'-end-side is 4 or more or the complementarity score S satisfies S > 5 and (B) for an ordinary primer, the complementarity score S satisfies S > 9, and when one of (A) and (B) is satisfied, the primers are determined to have non-specific amplification inducibility.

20. A non-transitory computer-readable recording medium on which the program according to claim 19 is recorded.
